# FASCICULE DE BREVET EUROPEEN

(11) **EP 1 014 890 B1**
(45) Date de publication et mention de la délivrance du brevet: **26.02.2003**
(21) Numéro de dépôt: 98945340.2
(22) Date de dépôt: 21.09.1998
(51) Int. Cl.: A61F 2/06

(54) **Ensemble pour l'angioplastie d'anévrisme comprenant une endoprothèse et un ballonnet d'expansion**
Anordnung für die Angioplastie bestehend aus einem Stent und einem Balloon zur Expansion
Assembly for aneurysm angioplasty comprising a stent and a balloon for expansion

(30) Priorité: 19.09.1997 FR 9711719
(43) Date de publication de la demande: 05.07.2000
(73) Titulaire: Bergeron, Patrice, 13009 Marseille (FR)
(72) Inventeur: Bergeron, Patrice, 13009 Marseille (FR)
(74) Mandataire: Breese, Pierre
(86) Numéro de dépôt international: FR9802014
(87) Numéro de publication internationale: WO99015102

(56) Documents cités:
- EP-A- 0 479 557
- EP-A- 0 779 062
- WO-A-96/28116

## Description

La présente invention concerne un ensemble pour le traitement d'un anévrisme vasculaire constitué par une endoprothèse et par un ballonnet pour l'expansion d'une telle prothèse.

Dans l'état actuel de la technique, on introduit au niveau de l'anévrisme une prothèse de forme cylindrique expansible. La demande PCT WO96/28116 décrit une prothèse présentant une extrémité biseautée.

Cette endoprothèse est formée par une armature expansible réalisée par exemple par un réseau de fils métalliques, recouvert par un matériau assurant l'étanchéité.

Elle assure la continuité du débit sanguin entre les parties vasculaires non atteintes par l'anévrisme, et prend appui en amont et en aval de l'anévrisme sur la paroi intérieure du collet.

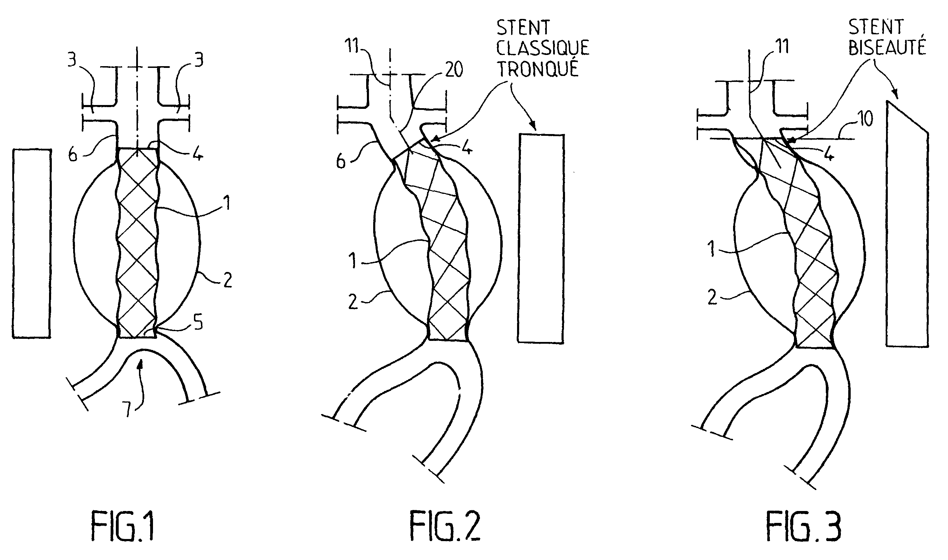

Les prothèses de l'art antérieur sont bien adaptées aux situations où le collet supérieur est droit et suffisamment long. La surface de contact de l'endoprothèse avec les parties vasculaires saines est alors suffisante pour éviter les fuites (voir figure 1).

Par contre, ces prothèses de l'art antérieur sont mal adaptées aux situations où le collet est court, ou oblique. Dans ces cas, le raccordement entre l'endoprothèse et la partie vasculaire saine est insuffisant, et des fuites peuvent se produire notamment au niveau du collet supérieur. Ces inconvénients ont tendance à s'aggraver avec le temps, en raison de glissements possibles de la prothèse, ou encore en cas d'aggravation de l'anévrisme.

Le but de l'invention est de remédier à cet inconvénient des prothèses de l'art antérieur.

La présente invention concerne un ensemble selon la revendication 1.

Selon une variante destinée à la réparation de l'anévrisme iliaque, on procède à l'expansion d'une endoprothèse iliaque à l'aide d'un ballonet de forme générale cylindrique prolongée à l'une des extrémités par une partie évasée.

L'invention sera mieux comprise à la lecture de la description qui suit, se référant aux dessins annexés où :
- la figure 1 représente une vue en coupe d'une solution de l'état de la technique ;
- la figure 2 représente une vue en coupe d'une telle solution, pour des cas où le collet est oblique ;
- la figure 3 représente une vue en coupe d'un anévrisme avec une endoprothèse ;
- la figure 4 représente une vue en coupe d'une endoprothèse implantée au niveau iliaque en cours d'expansion avec un ballonnet de l'art antérieur ;
- la figure 5 représente une vue en coupe d'une endoprothèse en cours d'expansion avec un ballonnet ;
- la figure 6 représente une vue en coupe d'une endoprothèse en cours d'expansion avec un ballonnet ; et
- la figure 7 représente une vue en coupe d'une endoprothèse en cours d'expansion avec un ballonnet selon une autre variante.

La prothèse (1) est formée par un tronçon tubulaire expansible dans sa partie supérieure. Elle présente une armature expansible en acier par exemple, noyée dans une enveloppe tubulaire en PTFE assurant l'étanchéité. L'endoprothèse (1) est amenée à travers l'aorte dans l'anévrisme (2), l'une des extrémités (4) de la prothèse étant positionnée dans le collet (6) juste en dessous des artères rénales (3), et l'autre extrémité (5) étant positionnée au-dessus de la bifurcation aortique (7).

A ce moment, on procède à l'expansion de l'endoprothèse (1) par mise sous pression d'un ballonnet. Le ballonnet est ensuite retiré, laissant l'endoprothèse (1) dans l'anévrisme (2). Le flux sanguin traverse au niveau-de l'anévrisme (2) l'endoprothèse (1), parfaitement étanche. L'anévrisme est alors préservé de la pression sanguine, et peut dans certains cas se résorber naturellement. Il arrive toutefois que le collet (6) soit court ou fortement oblique, comme représenté sur la figure 2. Dans ce cas, la zone de contact entre l'extrémité (4) de l'endoprothèse (1) selon l'état de la technique, et le coté extérieur de la courbure du collet (6) est très courte et induit un mauvais accrochage de l'endoprothèse (1). Si cette zone de contact est insuffisante, des fuites locales peuvent se produire. Ce risque est d'autant plus grand que l'anévrisme peut évoluer et augmenter de taille, réduisant alors encore plus la qualité du contact entre l'extrémité (4) et le collet (6).

La figure 3 représente une vue en coupe d'une endoprothèse. L'extrémité (4) de l'endoprothèse (1) est biseautée pour offrir une meilleure couverture de la paroi extérieure de la courbure et améliorer l'accrochage sur le collet. Les fuites sont de ce fait diminuées. L'axe (20) du collet de l'anévrisme n'est pas toujours dans la prolongation de l'axe médian (11) de l'aorte inter-rénale. Souvent, ces deux axes forment entre eux un angle de quelques degrés. Cet angle est compensé par une extrémité biseautée selon un angle tel que le plan frontal (10) soit perpendiculaire non pas à l'axe médian (20) du collet, et donc de l'endoprothèse, mais à l'axe médian (11) de l'aorte sus-rénale.

Le plan frontal (10) de l'extrémité de l'endoprothèse est perpendiculaire à l'axe médian (11) de l'aorte inter-rénale et forme avec l'axe longitudinal de l'endoprothèse un angle d'environ 30°.

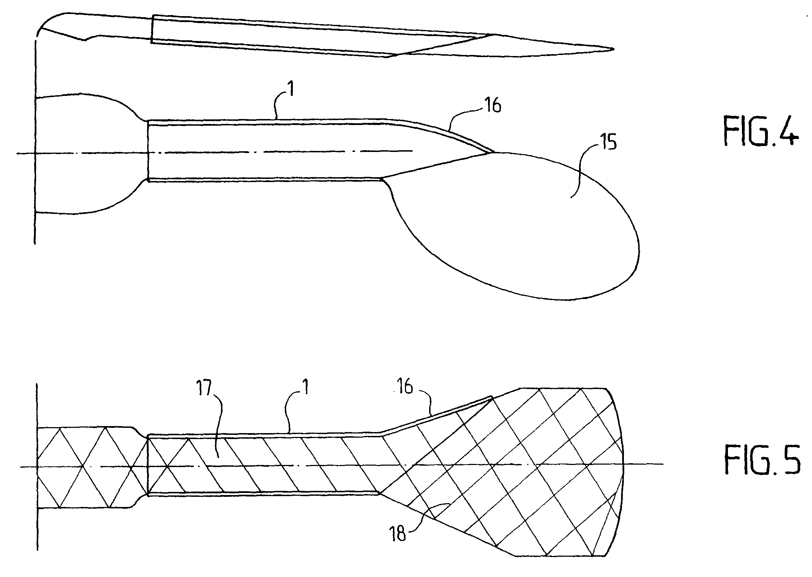

Les figures 4 et suivantes concernent l'implantation des endoprothèses biseautées implantées sur des artères iliaques, au niveau d'une bifurcation aortique (7). Le but est d'assurer le bon déploiement d'une endoprothèse lorsqu'elle se déploie dans l'aorte, c'est-à-dire qu'il n'a pas d'appui pour le ballon qui est dimensionné à la taille de l'iliaque et non de l'aorte.

Le brevet EP 0 779 062 décrit un ballonnet présentant une extrémité divergente, pour l'expansion d'instant tubulaire.

La figure 4 représente une vue en coupe d'une endoprothèse en cours d'expansion avec un ballonnet de l'art antérieur. On constate qu'utilisé avec une endoprothèse à l'extrémité biseautée, un tel ballonnet (15) ne permet pas le déploiement satisfaisant de l'extrémité biseautée (16). Le ballonnet se déforme par manque d'appui sur la partie biseautée (16) et provoque une courbure parasite de l'extrémité de l'endoprothèse.

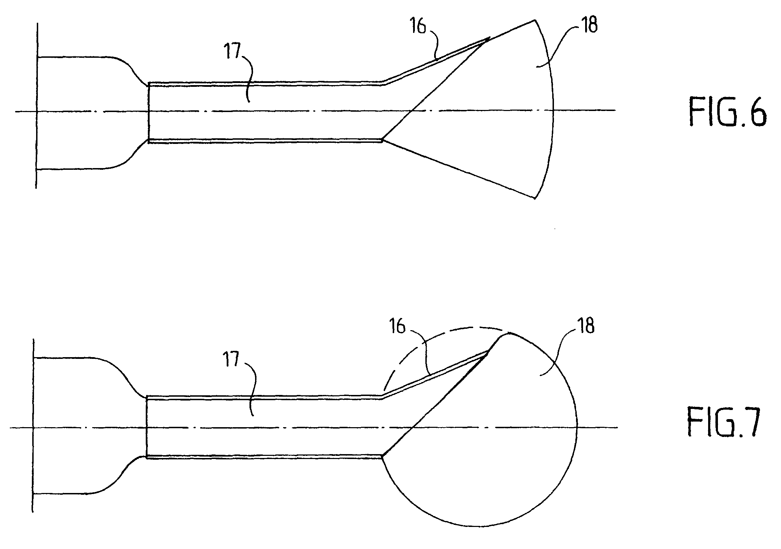

Les figures 5 et 6 représentent des vues en coupe d'une endoprothèse en cours d'expansion avec un ballonnet en prenant appui sur la paroi de l'artère iliaque opposée à l'artère iliaque traitée. Ce ballonnet présente une partie cylindrique (17) prolongé par une partie évasée (18) de forme conique. Cette partie évasée assure l'expansion optimale de l'extrémité biseautée de l'endoprothèse. Le ballonnet peut être armé comme représenté en figure 5 ou non armé comme représenté en figure 6.

La figure 7 représente une vue en coupe d'une endoprothèse en cours d'expansion avec un ballonnet selon une autre variante. La partie évasée (18) est de forme sphérique et assure un bon appui sur les parois intérieures de l'extrémité biseautée (16).

## Revendications

1. Ensemble pour le traitement d'un anévrisme vasculaire constitué par un ballonnet (15) d'expansion et par une endoprothèse (1) formée par une armature expansible recouverte par un matériau d'étanchéité et présentant une forme cylindrique dont l'extrémité (4) biseautée (16) réalise un plan frontal (10), **caractérisé en ce que** le ballonnet présente une forme générale cylindrique (17) prolongée à l'une de ses extrémités par une partie évasée (18).

2. Ensemble pour le traitement d'un anévrisme vasculaire selon la revendication 1, **caractérisé en ce que** ladite partie évasée (18) est armée.

3. Ensemble pour le traitement d'un anévrisme vasculaire selon la revendication 1, **caractérisé en ce que** ladite partie évasée (18) présente une forme conique.

4. Ensemble pour le traitement d'un anévrisme vasculaire selon la revendication 1 **caractérisé en ce que** ladite partie évasée (18) présente une forme sphérique.

## Claims

1. Assembly for treating a vascular aneurysm, consisting of an expansion balloon (15), and an endovascular stent (1) made of an expandable frame covered by a sealing material and having a cylindrical shape of which one end (4) which is bevelled (16) forms a frontal plane (10), **characterized in that** the balloon is of general cylindrical shape (17) extended at one of its ends by a flared part (18).

2. Assembly for treating a vascular aneurysm according to claim 1, **characterized in that** the said flared part (18) is reinforced.

3. Assembly for treating a vascular aneurysm according to claim 1, **characterized in that** said flared part (18) is of conical shape.

4. Assembly for treating a vascular aneurysm according to claim 1, **characterized in that** said flared part (18) is of spherical shape.

## Patentansprüche

1. Einheit zur Behandlung eines Gefä_aneurismas, die aus einem Erweiterungsballon (15) und einer Endoprothese (1) besteht, welche durch eine mit einem Abdichtungsstoff bedeckte, erweiterbare Einlage gebildet ist und eine zylindrische Form aufweist, deren abgeschrägtes (16) Ende (4) die Stirnfläche (10) bildet, **dadurch gekennzeichnet dass** der Ballon eine wesentlich zylindrische Form (17) aufweist, die an einem ihrer Enden durch einen erweiterten Teil (18) verlängert ist.

2. Einheit zur Behandlung eines Gefä_aneurismas nach Anspruch 1, **dadurch gekennzeichnet, dass** der besagte erweiterte Teil (18) verstärkt ist.

3. Einheit zur Behandlung eines Gefä_aneurismas nach Anspruch 1, **dadurch gekennzeichnet, dass** der besagte erweiterte Teil (18) kegelförmig ausgebildet ist.

4. Einheit zur Behandlung eines Gefä_aneurismas nach Anspruch 1, **dadurch gekennzeichnet, dass** der besagte erweiterte Teil (18) kugelförmig ausgebildet ist.
